# EUROPEAN PATENT APPLICATION

(11) **EP 4 008 266 A1**
(43) Date of publication of application: **08.06.2022**
(21) Application number: 21824279.0
(22) Date of filing: 17.09.2021
(51) Int. Cl.: A61B 8/00

(54) **ULTRASONIC DIAGNOSTIC DEVICE, ULTRASONIC IMAGE GENERATION METHOD AND STORAGE MEDIUM**

(30) Priority: 10.10.2020 CN 202011079552
(71) Applicant: Cloudminds Robotics Co., Ltd., Shanghai 200245 (CN)
(72) Inventor: LUO, Lei, Shanghai 200245 (CN)
(74) Representative: Renaudo, Adrien Hanouar
(86) International application number: PCT/CN2021/119039
(87) International publication number: WO 2022/073413

(57) **Abstract**

Some embodiments of the present disclosure relate to the field of ultrasonic waves, and disclose an ultrasonic diagnostic device, a method for generating an ultrasonic image, and a storage medium. The ultrasonic diagnostic device (10) in the present disclosure includes: an ultrasonic probe (101), an acquisition assembly (102) for a probe image, and a main control assembly (103) connected with the ultrasonic probe (101) and the acquisition assembly (102), respectively. The ultrasonic probe (101) is configured to continuously scan an object to be detected located on an operating table to acquire corresponding ultrasonic images, and transmit the ultrasonic images to the main control assembly (103); a photographing direction of the acquisition assembly (102) faces the operating table, and a photographing range covers the operating table; the acquisition assembly (102) is configured to acquire, when the ultrasonic probe (101) is operated, a probe image containing the ultrasonic probe (101) in real time; and the main control assembly (103) is configured to stitch, according to the probe image, the ultrasonic images, so as to generate an ultrasonic stereo image of the object to be detected. By adopting the implementation modes of the present disclosure, without professional knowledge, a user may acquire images accurately reflecting a lesion, and the usage difficulty of the ultrasonic diagnostic device is lowered.

## Description

### FIELD

The present disclosure relates to the technical field of ultrasonic waves, in particular to an ultrasonic diagnostic device, a method for generating an ultrasonic image, and a storage medium.

### BACKGROUND

For current ultrasonic diagnostic device, images acquired are 2D ultrasonic slice data. Professional doctors perform diagnosis on the basis of the 2D ultrasonic slice image. Saved data is also one or several 2D ultrasonic slice images.

However, a human body is a three-dimensional individual. Although a professional doctor may obtain, through techniques and experience, a 2D ultrasonic slice image that best reflects a disease, not all doctors can find a 2D ultrasonic slice image at the most ideal position in an ultrasonic inspection operation. For example, doctors working in town and village may rarely use or never use an ultrasound diagnostic device. If 2D ultrasonic slice images that accurately reflect lesions need to be acquired, a long time training of manipulation techniques with regard to each organ is required, and it is difficult for them to acquire correct 2D ultrasonic slice images only through remote guidance. Therefore, it is difficult to realize a remote diagnostic scenario of ultrasound, and ultrasound has not been able to be promoted on a large scale.

### SUMMARY

Some embodiments of the present disclosure aim to provide an ultrasonic diagnostic device, a method for generating an ultrasonic image, and a storage medium, so that without professional knowledge, a user may acquire an image accurately reflecting a lesion, and the difficulty in use of the ultrasonic diagnostic device is lowered.

The embodiments of the present disclosure provide an ultrasonic diagnostic device, including: an ultrasonic probe, an acquisition assembly for a probe image, and a main control assembly connected with the ultrasonic probe and the acquisition assembly, respectively; the ultrasonic probe is configured to continuously scan an object to be detected located on an operating table to acquire corresponding ultrasonic images, and transmit the ultrasonic images to the main control assembly; the acquisition assembly is arranged at a position above the operating table; a photographing direction of the acquisition assembly faces the operating table, and a photographing range covers the operating table; the acquisition assembly is configured to acquire, when the ultrasonic probe is operated, a probe image containing the ultrasonic probe in real time and transmit the probe image to the main control assembly; and the main control assembly is configured to stitch, according to the probe image, the ultrasonic images, so as to generate an ultrasonic stereo image of the object to be detected.

The embodiments of the present disclosure further provide a method for generating an ultrasonic image, including: when it is detected that an ultrasonic probe is operated, controlling an acquisition assembly located above an operating table to acquire a probe image containing the ultrasonic probe in real time; synchronously acquiring ultrasonic images that are generated by continuously scanning an object to be detected by the ultrasonic probe; and stitching, according to the probe image, the continuous ultrasonic images, so as to generate an ultrasonic stereo image of the object to be detected.

The embodiments of the present disclosure further provide a computer-readable storage medium which stores a computer program. The computer program, when executed by a processor, implements the method for generating an ultrasonic image. Compared with the existing art, the embodiments of the present disclosure have the advantages that the ultrasonic probe is configured to continuously scan the object to be detected located on the operating table, the acquisition assembly is arranged at the position above the operating table; the photographing direction of the acquisition assembly faces the operating table, and the photographing range covers the operating table; the acquisition assembly may acquire the probe image containing the ultrasonic probe in real time and transmit the acquired probe image to the main control assembly; and the main control assembly may acquire in real time the probe image and the ultrasonic images scanned by the corresponding probe and may thus stitch the continuously acquired ultrasonic images according to the probe image, so as to form the ultrasonic stereo image. Since the generated ultrasonic stereo image is a three-dimensional image displaying more details of the object to be detected, the user may read the ultrasonic stereo image as needed without professional ultrasonic operation knowledge, thus adding the usage scenario of the ultrasonic diagnostic device.

### BRIEF DESCRIPTION OF THE DRAWINGS

One or more embodiments are exemplified by the corresponding accompanying drawings. These exemplified descriptions do not constitute a limitation to the embodiments. Elements with the same reference numerals in the accompanying drawings are shown as similar elements. The drawings in the accompanying drawings do not constitute scaling restrictions unless otherwise stated.
FIG. 1 is a structural block diagram of an ultrasonic diagnostic device according to a first embodiment of the present disclosure;
FIG. 2 is a schematic diagram of an ultrasonic diagnostic device according to a second embodiment of the present disclosure;
FIG. 3 is a schematic diagram of a moving direction of an ultrasonic probe according to a second embodiment of the present disclosure;
FIG. 4 is a schematic diagram of arrangement of ultrasonic images according to a second embodiment of the present disclosure;
FIG. 5 is another schematic diagram of arrangement of ultrasonic images according to a second embodiment of the present disclosure;
FIG. 6 is a schematic diagram of an ultrasonic stereo image according to a second embodiment of the present disclosure;
FIG. 7 is a schematic diagram of an ultrasonic image of a specified section according to a second embodiment of the present disclosure;
FIG. 8 is a structural block diagram of an ultrasonic diagnostic device according to a third embodiment of the present disclosure;
FIG. 9 is a schematic diagram of an ultrasonic diagnostic device according to a third embodiment of the present disclosure;
FIG. 10 is a schematic diagram of a projection effect of an ultrasonic diagnostic device according to a third embodiment of the present disclosure;
FIG. 11 is a schematic effect diagram of a projection scanning path of an ultrasonic diagnostic device according to a third embodiment of the present disclosure;
FIG. 12 is a schematic diagram of arrangement of ultrasonic images according to a third embodiment of the present disclosure; and
FIG. 13 is a flowchart of a method for generating an ultrasonic image according to a fourth embodiment of the present disclosure.

### DETAILED DESCRIPTION OF EXAMPLE EMBODIMENTS

In order to make the objectives, technical solutions and advantages of the present disclosure clearer, some embodiments of the present disclosure is further described below in detail with reference to accompanying drawings and embodiments. Those of ordinary skill in the art can understand that in the various embodiments, many technical details are presented in order to make the present disclosure better understood by readers. However, the technical solutions claimed in the present disclosure can also be implemented without these technical details and various changes and modifications based on the embodiments. All the following embodiments are divided for the convenience of description and shall not constitute any restriction on the specific implementation modes of the present disclosure. All the embodiments can be combined and referenced with each other with no conflicts.

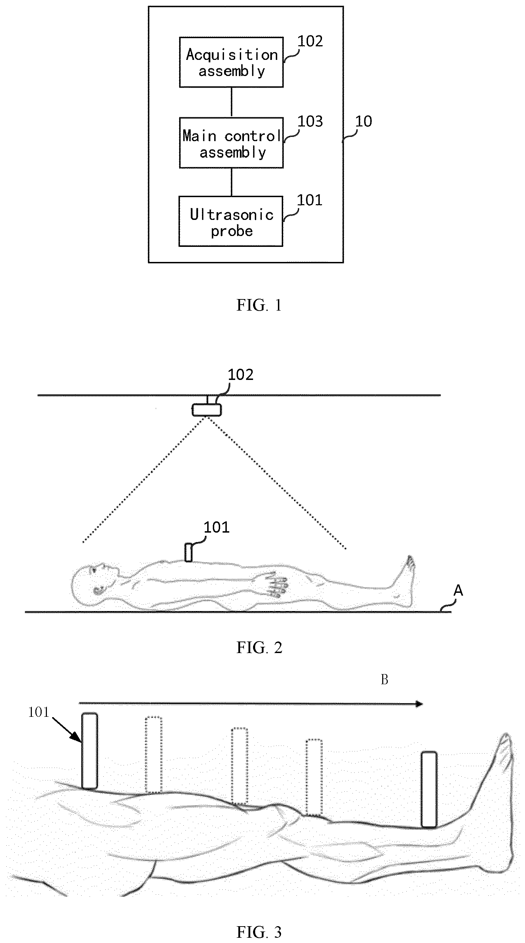

A first embodiment of the present disclosure provides an ultrasonic diagnostic device. The ultrasonic diagnostic device 10 includes: an ultrasonic probe 101, an acquisition assembly 102, and a main control assembly 103. The structural block diagram of the ultrasonic diagnostic device is shown in FIG. 1. The acquisition assembly 102 and the ultrasonic probe are connected with the main control assembly. The ultrasonic probe 101 is configured to continuously scan an object to be detected 30 located on an operating table 20 to acquire corresponding ultrasonic images, and transmit the ultrasonic images to the main control assembly 103; the acquisition assembly 102 is arranged at a position above the operating table 20; a photographing direction of the acquisition assembly 102 faces the operating table 20, and a photographing range covers the operating table 20; the acquisition assembly 102 is configured to acquire, when the ultrasonic probe 101 is operated, a probe image containing the ultrasonic probe 101 in real time and transmit the probe image to the main control assembly 103; and the main control assembly 103 is configured to stitch, according to the probe image, the ultrasonic images, so as to generate an ultrasonic stereo image of the object to be detected.

In the embodiments of the present disclosure, the ultrasonic probe is configured to continuously scan the object to be detected located on the operating table, the acquisition assembly is arranged at the position above the operating table; the photographing direction of the acquisition assembly faces the operating table, and the photographing range covers the operating table; the acquisition assembly may acquire the probe image containing the ultrasonic probe in real time and transmit the acquired probe image to the main control assembly; and the main control assembly may acquire in real time the probe image and the ultrasonic images scanned by the corresponding probe and may thus stitch the continuously acquired ultrasonic images according to the probe image, so as to form the ultrasonic stereo image. Since the generated ultrasonic stereo image is a three-dimensional image displaying more details of the object to be detected, the user may read the ultrasonic stereo image as needed without professional ultrasonic operation knowledge, thus adding the usage scenario of the ultrasonic diagnostic device.

A second embodiment of the present disclosure relates to an ultrasonic diagnostic device. The present embodiment is a specific introduction of the ultrasonic diagnostic device in the first embodiment. Disposing positions of all assemblies in the ultrasonic diagnostic device are introduced below in combination with FIG. 2.

As shown in FIG. 2, the operating table A is configured to assist the object to be detected in keeping a steady state. For example, the operating table A may be a sickbed, or the like. The operating table A with the object to be detected may be parallel to a horizontal plane. In an ultrasonic diagnostic process, the object to be detected may lie down on the operating table; the ultrasonic probe connected with the main control assembly 103 scans the object to be detected to obtain an ultrasonic image. The ultrasonic probe 101 may include a two-dimensional probe or a three-dimensional probe.

The acquisition assembly 102 is arranged at the position right above the operating table A. The acquisition assembly 102 is configured to photograph the ultrasonic probe in real time and may include at least one camera. The camera may be a 2D camera or a 3D camera. The photographing direction of the acquisition assembly 102 faces the operating table A, and the photographing range covers the whole operating table, so as to ensure that the acquisition assembly 102 is able to photograph the camera no matter how the ultrasonic probe 101 moves in the scanning process. In particular, the ultrasonic probe 101 and the acquisition assembly 102 are in communication connection with the main control assembly 103. The connection may be wireless connection or wired connection. The main control assembly 103 is not shown in FIG. 2. The main control assembly 103 may be arranged above the operating table A or may be arranged at other positions. The main control assembly 103 may include a processor, such as a computer device.

An operating process of the ultrasonic diagnostic device is as follows: when it is detected that the ultrasonic probe is operated, the main control assembly 103 controls the acquisition assembly to start to take images in real time; the ultrasonic probe 101 scans the obtained ultrasonic images and transmits them to the main control assembly in real time; and similarly, the acquisition assembly 102 transmits the probe image that is taken in real time to the main control assembly 103. The main control assembly 103 may synchronize the operation of the acquisition assembly 102 with the operation of the ultrasonic probe 101.

After touching the object to be detected, the ultrasonic probe 101 may send, to the main control assembly 103, prompt information indicating that the ultrasonic probe 101 is started. The main control assembly 103 may start the acquisition assembly 102 to perform real-time photographing after acquiring the prompt information.

It should be noted that a time tag that represents scanning time may be added, according to the chronological order of the scanning time, for each of the ultrasonic images. Similarly, a time tag that represents photographing time may be added for each probe image. The corresponding probe image in the ultrasonic image at the same time may be searched through the time tag.

In one example, the main control assembly 103 is specifically configured to: acquire a probe position and a probe angle of the ultrasonic probe in a preset three-dimensional spatial coordinate system according to the received probe image; place the ultrasonic image scanned by the current ultrasonic probe 101 on the probe position according to the probe angle in the three-dimensional spatial coordinate system; and if it is detected that the ultrasonic probe 101 completes the scanning, stitch each of the ultrasonic images located in the three-dimensional spatial coordinate system, so as to generate the ultrasonic stereo image.

Specifically, the three-dimensional spatial coordinate system may be preset. Any position in the three-dimensional spatial coordinate system may be taken as a corresponding probe position of the ultrasonic probe 101 in the three-dimensional spatial coordinate system, and any angle may also be taken as an initial probe angle in the preset three-dimensional spatial coordinate system.

There are various ways to acquire the probe position and the probe angle. Two ways to acquire the probe position and the probe angle are introduced below:
Way I: the main control assembly 103 may identify a position, in the probe image, of the ultrasonic probe 101 in the probe image after acquiring the probe image. Since the photographing direction of the acquisition assembly 102 is fixed, the camera in the acquisition assembly 102 is stationary, and the probe position of the current ultrasonic probe in the three-dimensional spatial coordinate system may be acquired according to the corresponding position of the ultrasonic probe 101 in the three-dimensional spatial coordinate system when the ultrasonic probe is started. The way to acquire the probe angle is similar.

For example, the initial probe position is set as (x0, y0, z0) in the three-dimensional spatial coordinate system, and the probe angle at the initial time t0 is set as (α0, β0, γ0); the ultrasonic probe in the probe image corresponding to t1 is identified, and the position of the ultrasonic probe in the probe image is acquired; the probe position of the probe at t1 may be determined according to a correspondence relationship between the position of the ultrasonic probe at the initial time t0 in the image and the initial probe position (x0, y0, z0); similarly, the probe angle of the ultrasonic probe at t1 may be determined according to a correspondence relationship between the angle of the ultrasonic probe at the initial time in the probe image relative to the object to be detected and the initial probe angle (α0, β0, γ0). The acquired probe position is an absolute position, and the probe angle is an absolute angle.

Way II: A relative position change of the ultrasonic probe may be acquired by means of a difference between the probe image at the current time and the probe image at the previous time, and the probe position of the ultrasonic probe at the current time may be determined according to the relative position change and absolute coordinates of the ultrasonic probe at the previous time. Similarly, an angle change of the ultrasonic probe may also be acquired, and the probe angle of the ultrasonic probe at the current time is determined according to the relative angle change and the probe angle of the ultrasonic probe at the previous time.

After the probe position and the probe angle of the ultrasonic probe are acquired, the ultrasonic image may be placed on the probe position according to the probe angle.

The ultrasonic probe continuously scans the object to be detected; each of the ultrasonic images is placed, in accordance with the probe position of the ultrasonic probe, in the three-dimensional spatial coordinate system; and after it is detected that the ultrasonic probe completes the scanning, each of the ultrasonic images in the three-dimensional spatial coordinate system may be stitched to generate the ultrasonic stereo image.

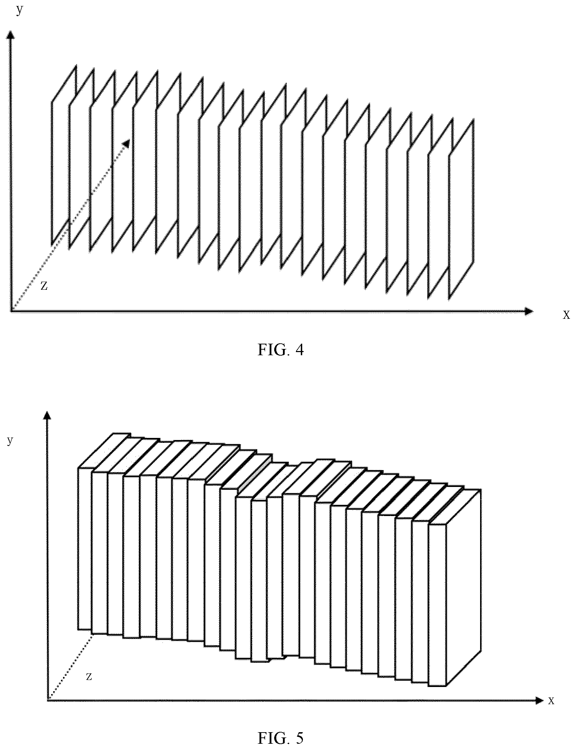

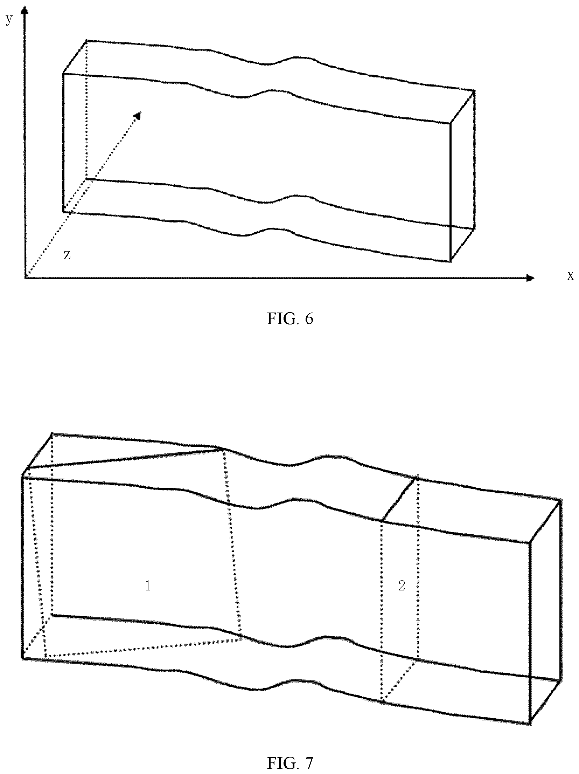

For example, the ultrasonic probe may scan a human leg according to a moving direction B as shown in FIG. 3. The human leg is an object to be detected. If the ultrasonic probe is a 2D probe, each of the ultrasonic images is placed in the three-dimensional spatial coordinate system according to the probe position of the ultrasonic probe. After it is detected that the ultrasonic probe completes the scanning, an ultrasonic image arrangement diagram as shown in FIG. 4 is formed. Each of the ultrasonic images is an optical coherence tomography. A section interval will also be different according to changes in a moving speed of the ultrasonic probe 101. The ultrasonic probe usually has a high sampling frequency, so an ultrasonic image arrangement diagram with a very small actual interval may be formed. The ultrasonic images arranged as shown in FIG. 4 are stitched to form the ultrasonic stereo image as shown in FIG. 6. If the moving speed of the ultrasonic probe is lower, a three-dimensional point cloud obtained is denser, and the formed ultrasonic stereo image is more accurate.

If the ultrasonic probe is a 3D probe, each frame of ultrasonic image has a certain width, and each scanned frame of ultrasonic image is a cubic slice with a certain width and is one three-dimensional optical coherence tomography of the leg, which is in a form as shown in FIG. 5. Since width information is acquired at each time, if the ultrasonic probe 101 moves slowly, there will be an image overlap part in two adjacent sampling operations, and the data of the overlap part shall be identical. Therefore, after each of the ultrasonic images is stitched, one stereo semipermeable dense point cloud graph is obtained, thus forming three-dimensional point cloud data and obtaining an ultrasonic stereo image of the leg, as shown in FIG. 6.

In one example, the ultrasonic diagnostic device 10 further includes: a display connected with the main control assembly. The display is configured to display the ultrasonic stereo image. The main control assembly 103 is further configured to slice, when an instruction that instructs a specified section to be display is detected, the ultrasonic stereo image, generate an ultrasonic image of the specified section, and transmit the ultrasonic image to the display.

Specifically, the display may be an ordinary display, or may be a holographic image display. The display displays the ultrasonic stereo image. If the user needs to observe the ultrasonic image of the specified section, the user may input information of the specified section through an input device. The main control assembly slices the ultrasonic stereo image after receiving the instruction, generates the ultrasonic image of the specified section, and transmits the ultrasonic image to the display. The display displays the ultrasonic image of the specified section. The specified section is a section 1 and a section 2 as shown in FIG. 7.

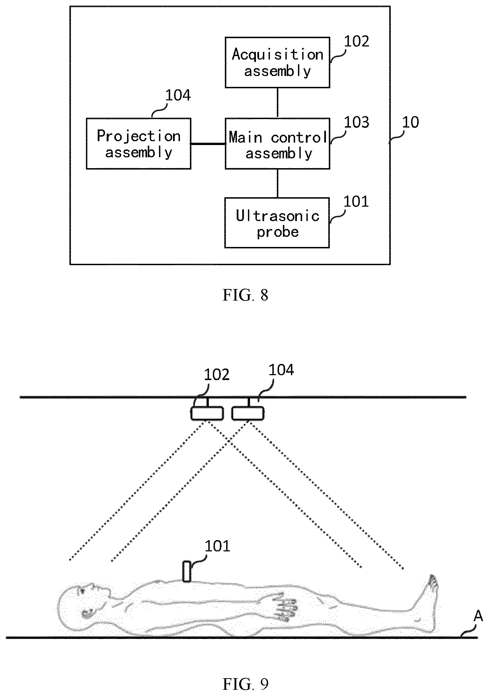

A third embodiment of the present disclosure relates to an ultrasonic diagnostic device. The present embodiment is an improvement of the first embodiment or the second embodiment. A main improvement is that the ultrasonic diagnostic device in this embodiment further includes a projection assembly 104. The structural block diagram of the ultrasonic diagnostic device is as shown in FIG. 8. Disposing positions of all assemblies in the ultrasonic diagnostic device are introduced below in combination with FIG. 9.

As shown in FIG. 9, the projection assembly 104 is arranged at a position above the operating table A. The projection assembly 104 is connected with the acquisition assembly 102 and the main control assembly 103, respectively. The position of the projection assembly 104 may be close to the acquisition assembly 102. The projection assembly 104 may be ordinary projection device. The projection assembly 104 may be connected to the main control assembly 103 in a wireless or wired manner.

The acquisition assembly 102 is further configured to photograph the object to be detected, acquire a first image, and transmit the first image to the main control assembly 103. The main control assembly 103 generates, according to the first image, a specified scanning region image containing a specified scanning region of the object to be detected; and the projection assembly 104 projects the specified scanning region image on the object to be detected.

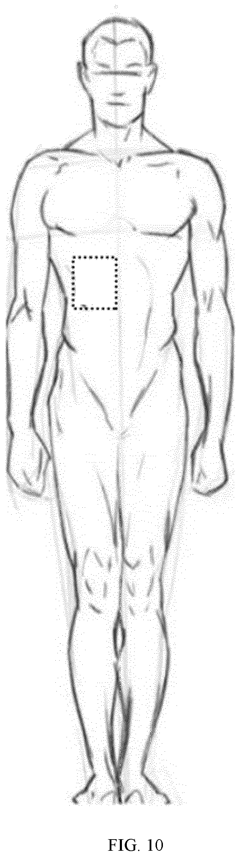

Specifically, the specified scanning region may be preset in the main control assembly 103. The specified scanning region may be an organ to be detected. A sensor may be arranged on the operating table A. The sensor is configured to detect whether there is an object to be detected on the operating table A and send a detection result to the main control assembly 103. After receiving indication information for indicating that there is an object to be detected on the operating table A, the main control assembly 103 starts the acquisition assembly 102 to photograph the object to be detected, and the first image is transmitted to the main control assembly 103. The main control assembly 103 generates, according to a position, in the image, of the object to be detected in the first image and the preset organ to be detected or a position to be detected, the specified scanning region image containing the specified scanning region of the object to be detected. The main control assembly 103 sends the specified scanning region image to the projection assembly 104. The projection assembly 104 projects the specified scanning region image on the object to be detected. The projection effect of the projection assembly is shown in FIG. 10. The square dotted line box is the specified scanning region image.

It should be noted that the main control assembly 103 may further set projection parameters of the projection assembly 104 according to the specified region image and the position of the object to be detected in the first image, so that corresponding specified scanning region images may be projected in case of different object to be detected.

It can be understood that the specified scanning region may also be an organ to be detected. The specified scanning region image may be an organ image corresponding to the object to be detected, and the organ image may be projected at the position of the organ to be detected of the object to be detected.

In one example, the main control assembly 103 is further configured to: generate, according to the specified scanning region, a scanning path image for indicating movement of the ultrasonic probe and send the scanning path image to the projection assembly 104; and the projection assembly 104 is further configured to project the scanning path image on the object to be detected.

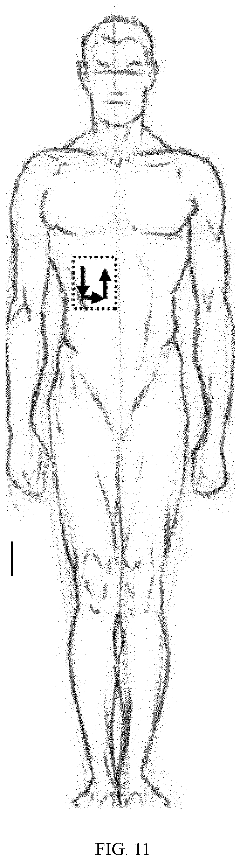

It is worth noting that the projected scanning path may be shown by the arrow in the dotted line box in FIG. 11. After a scanning path is projected, the user may control the ultrasonic probe to scan the specified scanning region according to the scanning path. In one example, the main control assembly 103 is further configured to: after it is detected that the ultrasonic probe 101 completes the scanning, if it is detected that an overlap part of a coverage region formed by all the ultrasonic images and the specified scanning region is smaller than the specified scanning region, output first error indication information; and/or, if it is detected that the ultrasonic images include a fuzzy image, output second error indication information.

Specifically, if it is detected that the ultrasonic probe 101 leaves a surface of the object to be detected, it is determined that the ultrasonic probe completes the scanning. Before the stitching of each of the ultrasonic images, it may be detected whether the overlap part between the coverage region formed by all the ultrasonic images and the specified scanning region is smaller than the specified scanning region; if yes, it is indicated that there is a region not scanned, and the first error indication information may be output. In addition, it may also be detected whether the acquired ultrasonic images include the fuzzy image; if yes, the second error indication information is output. The first error indication information includes an image of a missing region, and a sum of the missing region and the overlap part is greater than or equal to the specified scanning region; the second error indication information includes a photographing position for photographing the fuzzy image; the projection assembly is further configured to project the image of the missing region on the object to be detected; or, the projection assembly is further configured to project an image that represents the photographing position on the object to be detected.

The user may re-scan the missing region or the photographing position according to the image projected by the projection assembly, thereby obtaining an accurate ultrasonic image and improving the accuracy of the subsequently generated ultrasonic stereo image.

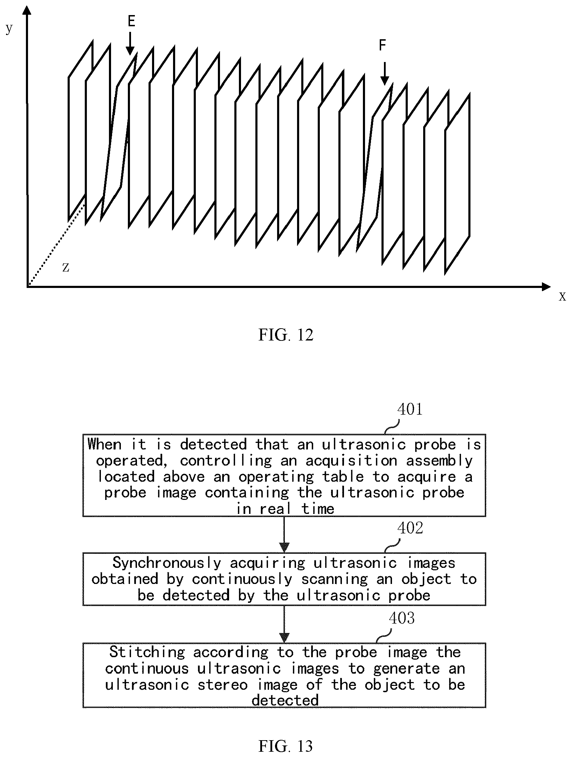

It should be noted that in the ultrasonic image arrangement diagram obtained by arrangement according to the probe angle, even if there is tilting, such as the ultrasonic image E and the ultrasonic image F as shown in FIG. 12, and if the scanning frequency is greater than a preset frequency, the tilted ultrasonic image may not be processed. If the scanning frequency is less than the preset frequency, it may instruct the scanning frequency to be increased to re-scan the specified scanning region.

The division of the steps of the various methods above is only for the clarity of description. When implemented, they can be combined into one step or some steps can be split into multiple steps, as long as they include the same logical relationship and all fall within the scope of protection of this patent. Adding insignificant modifications to algorithms or processes or introducing insignificant designs, but not changing the core designs of the algorithms and processes, falls within the scope of protection of this patent.

A fourth embodiment of the present disclosure relates to a method for generating an ultrasonic image, which is applied to an ultrasonic diagnostic device. The process is shown in FIG. 13.

At step 401: when it is detected that an ultrasonic probe is operated, an acquisition assembly located above an operating table is controlled to acquire a probe image containing the ultrasonic probe in real time.

Specifically, the acquisition assembly is arranged at the position above the operating table. The acquisition assembly may be a camera. A lens of the camera faces the operating table, and a photographing view of the camera covers the operating table. In particular, the acquisition assembly and the ultrasonic probe are in communication connection with the main control assembly. The communication connection may be wireless connection or wired connection.

When the ultrasonic probe contacts a surface of the object to be detected, it triggers the ultrasonic probe to be operated. When it is detected that the ultrasonic probe is operated, the acquisition assembly located above the operating table is controlled to acquire the probe image containing the ultrasonic probe in real time.

At step 402: ultrasonic images obtained by continuously scanning the object to be detected by the ultrasonic probe are continuously acquired.

Specifically, when the probe image is acquired, the ultrasonic image scanned by the ultrasonic probe at the corresponding time is synchronously acquired.

At step 403: the continuous ultrasonic images are stitched according to the pose data, so as to generate an ultrasonic stereo image of the object to be detected.

Specifically, a probe position and a probe angle of the ultrasonic probe in a preset three-dimensional spatial coordinate system are acquired according to the received probe image; the ultrasonic image scanned by the current ultrasonic probe is placed on the probe position according to the probe angle in the three-dimensional spatial coordinate system; and if it is detected that the ultrasonic probe completes the scanning, each of the ultrasonic images located in the three-dimensional spatial coordinate system is stitched, so as to generate the ultrasonic stereo image.

The three-dimensional spatial coordinate system may be preset. Any position in the three-dimensional spatial coordinate system may be taken as an initial probe position in the three-dimensional spatial coordinate system when the ultrasonic probe is started, and any angle may also be taken as an initial probe angle in the preset three-dimensional spatial coordinate system.

There are various ways to acquire the probe position and the probe angle. Two ways to acquire the probe position and the probe angle are introduced below:
Way I: the main control assembly 103 may identify a position, in the probe image, of the ultrasonic probe 101 in the probe image after acquiring the probe image. Since the photographing direction of the acquisition assembly 102 is fixed, the camera in the acquisition assembly 102 is stationary, and the probe position of the current ultrasonic probe in the three-dimensional spatial coordinate system may be acquired according to the corresponding position of the ultrasonic probe 101 in the three-dimensional spatial coordinate system when the ultrasonic probe is started. The way to acquire the probe angle is similar.

For example, the initial probe position is set as (x0, y0, z0) in the three-dimensional spatial coordinate system, and the probe angle at the initial time t0 is set as (α0, β0, γ0); the ultrasonic probe in the probe image corresponding to t1 is identified, and the position of the ultrasonic probe in the probe image is acquired; the probe position of the probe at t1 may be determined according to a correspondence relationship between the position of the ultrasonic probe at the initial time t0 in the image and the initial probe position (x0, y0, z0); similarly, the probe angle of the ultrasonic probe at t1 may be determined according to a correspondence relationship between the angle of the ultrasonic probe at the initial time in the probe image relative to the object to be detected and the initial probe angle (α0, β0, γ0). The acquired probe position is an absolute position, and the probe angle is an absolute angle.

Way II: A relative position change of the ultrasonic probe may be acquired by means of a difference between the probe image at the current time and the probe image at the previous time, and the probe position of the ultrasonic probe at the current time may be determined according to the relative position change and absolute coordinates of the ultrasonic probe at the previous time. Similarly, an angle change of the ultrasonic probe may also be acquired, and the probe angle of the ultrasonic probe at the current time is determined according to the relative angle change and the probe angle of the ultrasonic probe at the previous time.

After the probe position and the probe angle of the ultrasonic probe are acquired, the ultrasonic image may be placed on the probe position in accordance with the probe angle.

The ultrasonic probe continuously scans the object to be detected; each of the ultrasonic images is placed, in accordance with the probe position of the ultrasonic probe, in the three-dimensional spatial coordinate system; and after it is detected that the ultrasonic probe completes the scanning, each of the ultrasonic images in the three-dimensional spatial coordinate system may be stitched to generate the ultrasonic stereo image. The main control assembly in the ultrasonic diagnostic device may include a memory and a processor. The memory is in communication connection with the at least one processor. The memory stores an instruction that may be executed by the at least one processor. The instruction is executed by the at least one processor to cause the at least one processor to be able to implement the above method for generating an ultrasonic image.

The memory and the processor are connected by a bus. The bus may include any number of interconnected buses and bridges. The bus links one or more processors with various circuits of the memory together. The bus may also link various other circuits such as peripherals, voltage regulators and power management circuits, which are well known in the art and, therefore, will not be further described herein. A bus interface provides an interface between the bus and a transceiver. The transceiver may be one element or a plurality of elements, such as a plurality of receivers and transmitters to provide units for communicating with various other devices on a transmission medium. Data processed by the processor is transmitted on a wireless medium through an antenna, and further, the antenna also receives the data and transmits the data to the processor.

The processor is responsible for managing the bus and performing general processing, and can also provide various functions including timing, peripheral interfaces, voltage regulation and power management, and other control functions. The memory can be used to store data used by the processor when performing operations.

A fifth embodiment of the present disclosure relates to a computer-readable storage medium which stores a computer program. The computer program, when executed by a processor, implements the method for generating an ultrasonic image.

Those skilled in the art can understand that all or part of the steps in the method of the above embodiment can be completed by a program that instructs related hardware. This program is stored in a storage medium and includes a number of instructions configured to enable one device (which may be a single-chip microcomputer, a chip, and the like) or a processor to execute all or part of the steps of the methods described in the various embodiments of the present disclosure. The aforementioned storage media include: a USB flash disk, a mobile hard disk, a read-only memory (ROM), a random access memory (RAM), a magnetic disk or an optical disk, and other media that can store program codes.

Those of ordinary skill in the art can understand that the above-mentioned implementation modes are specific embodiments for realizing the present disclosure, and in actual applications, various changes can be made in form and details without departing from the spirit and scope of the present disclosure.

## Claims

1. An ultrasonic diagnostic device, comprising an ultrasonic probe, an acquisition assembly, and a main control assembly connected with the ultrasonic probe and the acquisition assembly, respectively; wherein,
the ultrasonic probe is configured to continuously scan an object to be detected located on an operating table to acquire corresponding ultrasonic images, and transmit the ultrasonic images to the main control assembly;
the acquisition assembly is arranged at a position above the operating table; and a photographing direction of the acquisition assembly faces the operating table, and a photographing range covers the operating table;
the acquisition assembly is configured to acquire, when the ultrasonic probe is operated, a probe image containing the ultrasonic probe in real time and transmit the probe image to the main control assembly;
the main control assembly is configured to stitch, according to the probe image, the ultrasonic images, so as to generate an ultrasonic stereo image of the object to be detected.

2. The ultrasonic diagnostic device according to claim 1, wherein the main control assembly is specifically configured to:
acquire a probe position and a probe angle of the ultrasonic probe in a preset three-dimensional spatial coordinate system according to the received probe image;
place the ultrasonic image scanned by the current ultrasonic probe on the probe position in accordance with the probe angle in the three-dimensional spatial coordinate system; and
if it is detected that the ultrasonic probe completes a scanning, stitch each of the ultrasonic images located in the three-dimensional spatial coordinate system, so as to generate the ultrasonic stereo image.

3. The ultrasonic diagnostic device according to claim 1 or 2, wherein the ultrasonic diagnostic device further comprises a projection assembly arranged at a position above the operating table; the projection assembly is connected with the acquisition assembly and the main control assembly, respectively;
the acquisition assembly is further configured to photograph the object to be detected, to acquire a first image, and transmit the first image to the main control assembly;
the main control assembly generates, according to the first image, a specified scanning region image containing a specified scanning region of the object to be detected;
the projection assembly projects the specified scanning region image on the object to be detected.

4. The ultrasonic diagnostic device according to any one of claims 1 to 3, wherein the main control assembly is further configured to:
after it is detected that the probe main body completes the scanning, if an overlap part between a coverage region formed by all the acquired ultrasonic images and the specified scanning region is smaller than the specified scanning region, output first error indication information; and/or,
if it is detected that the ultrasonic images comprise a fuzzy image, output second error indication information.

5. The ultrasonic diagnostic device according to claim 4, wherein the first error indication information comprises an image of a missing region, wherein a sum of the missing region and the overlap part is greater than or equal to the specified scanning region;
the second error indication information comprises a photographing position for photographing the fuzzy image;
the projection assembly is further configured to project the image of the missing region on the object to be detected; or, the projection assembly is further configured to project an image representing the photographing position on the object to be detected.

6. The ultrasonic diagnostic device according to any one of claims 1 to 5, wherein the main control assembly is further configured to:
generate, according to the specified scanning region, a scanning path image for indicating movement of the ultrasonic probe and send the scanning path image to the projection assembly;
the projection assembly is further configured to project the scanning path image on the object to be detected.

7. The ultrasonic diagnostic device according to any one of claims 1 to 6, wherein the ultrasonic probe comprises a two-dimensional probe or a three-dimensional probe.

8. The ultrasonic diagnostic device according to any one of claims 1 to 7, wherein the ultrasonic diagnostic device further comprises a display connected with the main control assembly;
the display is configured to display the ultrasonic stereo image;
the main control assembly is further configured to slice, when an instruction instructing a specified section to be display is detected, the ultrasonic stereo image, to generate an ultrasonic image of the specified section, and transmit the ultrasonic image to the display.

9. A method for generating an ultrasonic image, comprising:
when it is detected that an ultrasonic probe is operated, controlling an acquisition assembly located above an operating table to acquire a probe image containing the ultrasonic probe in real time;
synchronously acquiring ultrasonic images obtained by continuously scanning an object to be detected by the ultrasonic probe; and
stitching, according to the probe image, the continuous ultrasonic images, so as to generate an ultrasonic stereo image of the object to be detected.

10. A computer-readable storage medium, storing a computer program, wherein the computer program, when executed by a processor, implements the method for generating an ultrasonic image according to claim 9.
